(19) **Europäisches Patentamt / European Patent Office / Office européen des brevets**

(11) **EP 1 455 712 B2**

(12) # NEW EUROPEAN PATENT SPECIFICATION
### After opposition procedure

(45) Date of publication and mention
of the opposition decision:
**09.03.2016 Bulletin 2016/10**

(45) Mention of the grant of the patent:
**09.08.2006 Bulletin 2006/32**

(21) Application number: **02793608.7**

(22) Date of filing: **09.12.2002**

(51) Int Cl.:
***A61F 13/15*** *(2006.01)*

(86) International application number:
**PCT/SE2002/002260**

(87) International publication number:
**WO 2003/063746 (07.08.2003 Gazette 2003/32)**

(54) **ABSORBENT ARTICLE**

SAUGFÄHIGER ARTIKEL

ARTICLE ABSORBANT

(84) Designated Contracting States:
**AT BE BG CH CY CZ DE DK EE ES FI FR GB GR IE IT LI LU MC NL PT SE SI SK TR**

(30) Priority: **18.12.2001 SE 0104268**

(43) Date of publication of application:
**15.09.2004 Bulletin 2004/38**

(73) Proprietor: **SCA Hygiene Products AB
405 03 Göteborg (SE)**

(72) Inventors:
- **DREVIK, Solgun
  S-435 35 Mölnlycke (SE)**
- **GUSTAVSSON, Anette
  S-421 67 Västra Frölunda (SE)**

(74) Representative: **Valea AB
Box 1098
405 23 Göteborg (SE)**

(56) References cited:

| | |
|---|---|
| **EP-A1- 0 518 340** | **EP-A1- 0 875 224** |
| **EP-A1- 1 040 800** | **WO-A1-00/10500** |
| **WO-A1-00/56257** | **WO-A1-96/19173** |
| **WO-A1-99/12734** | **SE-B- 384 786** |
| **US-A- 3 403 681** | **US-A- 3 886 941** |
| **US-A- 4 341 217** | **US-A- 4 681 793** |
| **US-A- 4 701 237** | **US-A- 5 614 283** |
| **US-A- 5 873 963** | **US-B1- 6 238 379** |

- **Chambers Science and Technology Dictionary (1988), p.189,725.**

EP 1 455 712 B2

**Description**

TECHNICAL FIELD

The invention relates to an absorbent article such as a diaper, a pant diaper, an incontinence pad, a panty liner, a micro panty liner, a sanitary towel or the like, having a first essentially liquid permeable surface layer, an essentially liquid impermeable backing layer and, located between said liquid permeable surface layer and said liquid impermeable backing layer, an absorbent body comprising at least one layer.

BACKGROUND ART

When an absorbent article such as a diaper, a pant diaper, an incontinence pad, an incontinence product with a belt, a panty liner, a micro panty liner, a sanitary towel or the like is used, part of the skin is covered in all cases. This means that it is more difficult for the skin to perspire, that is to say to breathe, often with the result that it becomes warm and moist inside the absorbent article. The problem becomes even more marked when the absorbent article has been used, that is to say exposed to motions, urine, menstrual fluid or the like.

Apart from this feeling unpleasant and uncomfortable for the wearer, there are also direct hygienic aspects to be considered. A warm and moist environment is a good breeding ground for bacteria, fungi and the like. This becomes particularly marked in absorbent articles which have been exposed to motions, urine or menstrual fluid (or mixtures thereof), where a warm moist environment together with the bacterial variety which originates from the bodily discharges can lead to active growth of undesirable microorganisms with consequences such as unpleasant odours, skin irritation, rashes, itching and the like.

Attempts to bring about breathability in absorbent articles have previously been made by inter alia using what are known as breathable backings. Document EP 1,040,800 A1, for example, describes a backing layer made of plastic film with perforated holes. However, this document does not offer an adequate solution to the problem as only those parts of the absorbent article which lie next to the backing layer can benefit from the breathable backing and the increased airiness it is said to afford.

Document WO 00/10500 describes an absorbent article with what is known as a ventilation ply. The ventilation ply has large straight cylindrical holes which are intended to create breathable zones in the absorbent article. This means that a large part of the absorption capacity in these parts of the core is lost. In addition to this, the holes are moreover occluded by other layers and therefore provide only a limited effect in the form of airiness.

Document EP0518340 A1 discloses the preamble of claim 1.

A need therefore exists not only to bring about breathability in part of the absorbent article but also to create a fully breathable absorbent article in a sophisticated manner, which can transport moist air away from broadly speaking the whole of the absorbent article while retaining leakproofness.

DISCLOSURE OF THE INVENTION

By means of the present invention, an absorbent article in accordance with claim 1 has been produced, which article essentially eliminates the problems of previously known such articles.

Essentially cone-shaped cavities are described below. In this connection, the term essentially cone-shaped cavity also means various pyramid-like and funnel-like shapes. Essentially cone-shaped cavities also mean, in addition to purely conical cavities, those cavities which have more sides than a pyramidal shape. It is pointed out that the more sides the essentially cone-shaped cavity has, the closer it comes to a pure cone shape.

By making essentially cone-shaped cavities through all of the absorbent body, an airy absorbent article is produced without appreciably impairing the leakage security. The absorption capacity in the ventilated absorbent body can even be regenerated to a certain extent by virtue of the liquid drying out. The precondition for this form of regeneration of the absorption capacity is that the absorbent article is very airy, that is to say breathable, because it should dry out as quickly as possible.

The liquid which the wearer discharges when wearing the article will not run down into the essentially cone-shaped cavities on account of the capillary forces in the tips of the cavities, which tips face the liquid permeable surface layer. As the cavities are essentially cone-shaped, the liquid will initially tend to remain in the tip of the cone, then being absorbed by the absorbent body and thus not running through the article.

The capillary force to which a liquid is subjected is usually described approximately by Laplace's equation.

Laplace's equation gives an idea of the net force which influences the liquid to go from a larger capillary to a smaller capillary.

$$\Delta P = p_A - p_R = \gamma \left( \frac{2 \cdot \cos\theta_A}{r_A} - \frac{2 \cdot \cos\theta_R}{r_R} \right)$$

$p_A$ = the capillary force in the smaller part
$p_R$ = the capillary force in the larger part
y = the surface tension
$\theta$ = the wetting angle
r = the radius of the capillary
where A is the smaller capillary and R the larger. It is therefore possible to demonstrate that the liquid will not run in the direction from the tip to the wider opening of a cone-shaped cavity but will stay in the tip of the essentially cone-shaped cavity. Through the base of the essentially cone-shaped cavity, air can easily enter large parts of the absorbent body and in this way dry or simply air the absorbent article and thus also the skin which is covered (occluded) by the absorbent article.

The essentially cone-shaped cavities extend through the whole of the absorbent body. This gives a high degree of breathability and makes it possible for the absorbent article and its absorbent core to transport away moisture which might be present in the various plies of the absorbent core.

According to another non-claimed embodiment, the absorbent body has essentially cone-shaped cavities arranged in specific zones of the absorbent body. In cases when extra breathability is desired in specific zones, this can be brought about by only certain parts of the absorbent article being perforated. For example, the whole article apart from the wetting zone can be perforated. In this connection, wetting zone means that part of the absorbent article which is expected to receive liquid first when, for example, urination or menstruation takes place. It is also possible to arrange the cavities in patterns such as stripes, to arrange the essentially cone-shaped cavities with different density (that is to say number of holes per unit of area), to use different sizes, for example two or more sizes, for the essentially cone-shaped cavities, or the like. It is also possible to make different zones with essentially cone-shaped cavities in combination with zones of pyramid-shaped and/or funnel-shaped cavities.

In a further embodiment, the envelope surface of the essentially cone-shaped cavities consists entirely or partly of the backing layer. When the envelope surface consists entirely or partly of the backing layer, it acquires the surface characteristics of the latter, which can be very advantageous. It is possible with a hydrophobic backing layer, for example, to obtain a hydrophobic envelope surface which assists the essentially cone-shaped cavities in not allowing the essentially hydrophilic liquid to pass through.

In a similar manner, it is possible to change the surface characteristics of the envelope surface in the essentially cone-shaped cavities by treating the backing layer before it is perforated and the essentially cone-shaped cavities are formed. The backing layer can also be treated during perforation itself by, for example, the roller which is used for perforation being coated with an agent which is transferred to the backing layer during perforation. It is then the inside of the essentially cone-shaped cavity which is partly treated by virtue of that side of the backing layer which faces away from the absorbent body coming into contact with the perforation roller.

The backing layer can also be treated on the side lying next to the absorbent body, and it is also possible to treat both sides.

Treating the backing layer may be desirable because the essentially cone-shaped cavities extend into the absorbent body and come into contact with it. The interior of the absorbent body is therefore exposed to the surface of the backing layer, and a number of possibilities are thus afforded for supplying substances or the like from the backing layer to the absorbent body. A number of possible treatments of the backing layer may be mentioned, for example pH treatment for pH control or treatment in order to provide a more hydrophobic surface. In certain cases, treatment in order to provide a less hydrophobic surface or a hydrophilic surface may be desirable. It is also possible to use an indicator treatment in order to provide an indication of various conditions such as temperature, moisture content, pH, enzyme activity or the like. The backing layer can also be treated in order to add bacteria to the article. Examples of suitable bacteria are lactobacteria, but it is also possible to carry out treatment for odour inhibition or a number of similar functions.

According to an embodiment, the envelope surface of the essentially cone-shaped cavities consists entirely or partly of a separate layer, for example a plastic film or a non-woven, located between the absorbent body and the liquid impermeable backing layer. Such a layer means that the essentially cone-shaped cavities are not so clearly visible from the outside, which can be psychologically important for a wearer to have confidence in the absorbent article. It also means that it is possible to give the cavities characteristics which perhaps cannot be achieved with a backing layer, or which are not desirable in a backing layer. The envelope surface of the essentially cone-shaped cavities can also be at least partly hydrophobic.

There are a number of different ways of producing a breathable absorbent article such as a diaper, a pant diaper, an incontinence pad, a panty liner, a micro panty liner, a sanitary towel or the like. The production methods disclosed herein are not part of the claimed invention. For example, the production can take place as follows:

i) an absorbent body comprising at least one layer and having a first and a second surface is located on a backing layer with the second surface of the absorbent body towards the backing layer;

ii) the backing layer and at least part of the absorbent body are perforated so that the backing layer and at least part of the absorbent body have cavities which are essentially cone-shaped and extend in a tapering manner from the backing layer and the second surface of the absorbent body towards the first surface of the absorbent body;

iii) a liquid permeable surface layer is located on the first surface of the perforated absorbent body so that the absorbent body lies between the liquid permeable surface layer and the perforated backing layer, and

iv) the liquid permeable surface layer and the backing layer are joined together.

According to an embodiment of the process, the backing layer and the absorbent body are perforated so that the backing layer and the absorbent body have cavities running all the way through which are essentially cone-shaped and extend in a tapering manner from the backing layer to the first surface of the absorbent body.

Another way of producing a breathable absorbent article such as a diaper, a pant diaper, an incontinence pad, a panty liner, a micro panty liner, a sanitary towel or the like is as follows:

i) an absorbent body comprising at least one layer and having a first and a second surface is located on a backing layer with the second surface of the absorbent body towards the backing layer;

ii) a liquid permeable surface layer is located on the first surface of the absorbent body so that the absorbent body lies between the surface layer and the backing layer;

iii) the backing layer and at least part of the absorbent body are perforated so that the backing layer and at least part of the absorbent body have cavities which are essentially cone-shaped and extend from the backing layer and the second surface of the absorbent body in a tapering manner towards the first surface of the absorbent body, and

iv) the liquid permeable surface layer and the backing layer are joined together.

In an embodiment of the process just described, the backing layer and the absorbent body are perforated so that the backing layer and the absorbent body have cavities running all the way through which are essentially cone-shaped and extend in a tapering manner from the backing layer to the first surface of the absorbent body.

Another way of producing a breathable absorbent article such as a diaper, a pant diaper, an incontinence pad, a panty liner, a micro panty liner, a sanitary towel or the like is as follows:

i) an absorbent body comprising at least one layer and having a first and a second surface is perforated so that at least part of the absorbent body has cavities which are essentially cone-shaped and extend from the second surface of the absorbent body in a tapering manner towards the first surface of the absorbent body;

ii) the second surface of the absorbent body is located against a liquid impermeable backing layer;

iii) a liquid permeable surface layer is located on the first surface of the absorbent body so that the absorbent body lies between the liquid permeable surface layer and the backing layer, and

iv) the liquid permeable surface layer and the backing layer are joined together.

In an embodiment of the process just described, the absorbent body is perforated so that the absorbent body has cavities running all the way through which are essentially cone-shaped and extend in a tapering manner from the second surface of the absorbent body to the first surface of the absorbent body.

Another way of producing a breathable absorbent article such as a diaper, a pant diaper, an incontinence pad, a panty liner, a micro panty liner, a sanitary towel or the like is as follows:

i) an absorbent body comprising at least one layer and having a first and a second surface is provided;

ii) the second surface of the absorbent body is located against a liquid impermeable backing layer;

iii) a liquid permeable surface layer is located on the first surface of the absorbent body so that the absorbent body lies between the surface layer and the backing layer, and the liquid permeable surface layer and the backing layer are joined together, and

iv) the backing layer and at least part of the absorbent body are perforated so that the backing layer and at least part of the absorbent body are provided with cavities which are essentially cone-shaped and extend in a tapering manner from the backing layer and the second surface of the absorbent body towards the first surface of the absorbent body.

In an embodiment of the process just described, the backing layer and the absorbent body are perforated so that the backing layer and the absorbent body have cavities running all the way through which are essentially cone-shaped and extend in a tapering manner from the backing layer and the second surface of the absorbent body to the first surface of the absorbent body.

In all the methods just mentioned above for producing an absorbent article according to the invention, the perforation can be carried out by means of, for example, a hot-needle perforator, a cold-needle perforator, by punching, by means of tapered cutting tools, a laser, a stamp perforator, milling or the like.

BRIEF DESCRIPTION OF FIGURES

The invention will be described in greater detail below with reference to figures shown in the accompanying drawings, in which

Fig. 1          shows a panty liner;

Fig. 2a        shows part of a section along the line II-II through an embodiment of the panty liner in Fig. 1 which is not part of the claimed invention;

Fig. 2b        shows part of a section along the line II-II through the panty liner in Fig. 1 for an alternative embodiment of the invention;

Fig. 2c        shows part of a section along the line II-II through the panty liner in Fig. 1 for an embodiment which is not part of the claimed invention;

Fig. 2d        shows part of a section along the line II-II through the panty liner in Fig. 1 for an alternative embodiment of the invention;

Fig. 2e        shows part of a section along the line II-II through the panty liner in Fig. 1 for an alternative embodiment of the invention;

Figs 3a-b      show essentially cone-shaped volumes, and

Fig. 3c        shows an X-Y diagram in which a rotation function with associated delimitations is plotted.

MODES FOR CARRYING OUT THE INVENTION

The invention can be applied to a number of different absorbent articles, for example diapers, pant diapers, incontinence pads, incontinence products with a belt, panty liners, micro panty liners, sanitary towels or the like. However, only a panty liner will be described below.

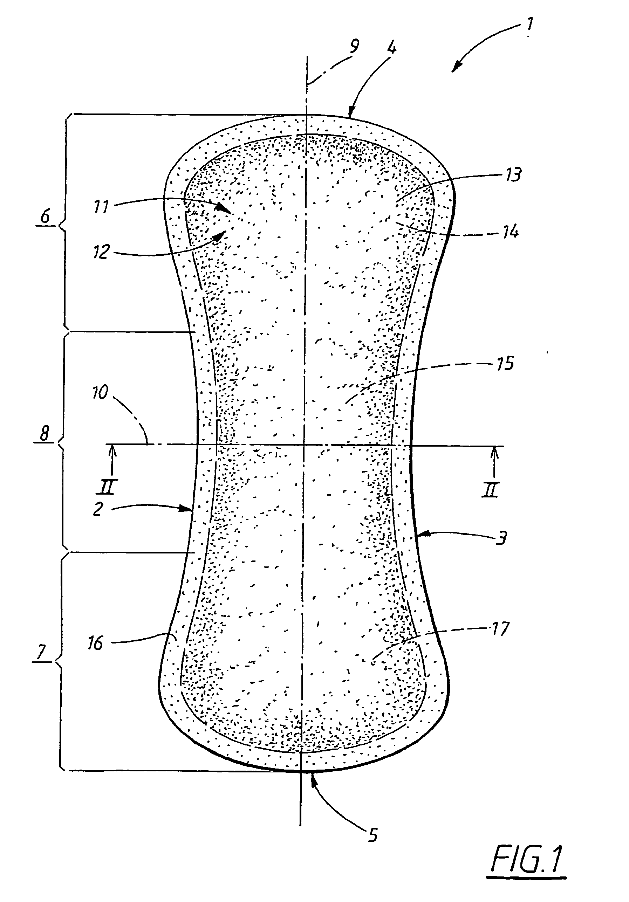

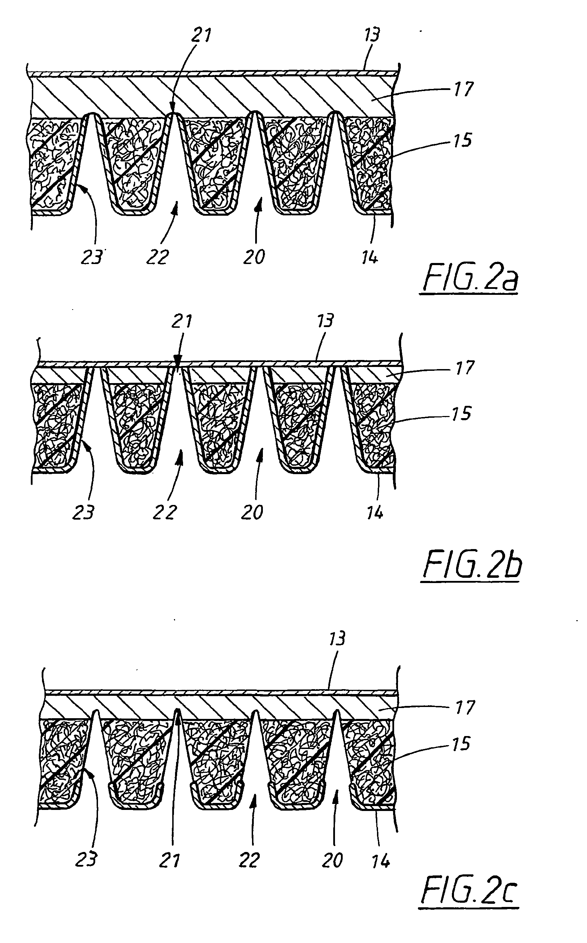

The panty liner 1 in Figures 1 and 2a-e has an essentially elongate shape with a longitudinal direction and a transverse direction and has two long sides 2, 3, two short sides 4, 5, a first end portion 6, a second end portion 7 and a crotch portion 8 located between the first end portion 6 and the second end portion 7, a longitudinal centre line 9 extending in the longitudinal direction of the panty liner and a transverse centre line 10 extending in the transverse direction of the panty liner. Centre line means a line which runs in the longitudinal or transverse direction and is located at an equal distance from the long sides 2, 3 or, respectively, the short sides of the panty liner 1. The panty liner 1 has a top side 11 which is intended to face the wearer during use and an underside 12 intended to face away from the wearer.

The panty liner 1 comprises a liquid permeable surface layer 13 arranged on the top side 11 so that it faces the wearer during use, and a liquid-barrier backing layer 14 on the underside 12. Arranged between the surface layer 13 and the backing layer 14 is an absorbent body 15. The surface layer 13 and the backing layer 14 are suitably connected to the absorbent body 15 by adhesive and, during manufacture of the panty liner, the various layers can be pressed together during adhesive bonding by an embossed roller, for example around the edge 16 of the panty liner. It is also possible to emboss patterns into the article during manufacture. Inside the edge 16, the article is of essentially uniform thickness, which means that it has a plane shape in order for good contact with the outer parts of the genital area of the wearer to be obtained.

On the underside 12 of the panty liner, there may be fastening means (not shown in the figure) in the form of, for example, pressure-sensitive adhesive. The adhesive can cover the entire underside, be applied in parallel strands along the underside or be applied to the underside in another suitable pattern such as, for example, diamond patterns or dots. It is nevertheless important not to apply so much adhesive that the panty liner is no longer breathable. A removable protective layer can be arranged over the adhesive. The protective layer is removed by the wearer before fitting the panty liner in the underwear of the wearer. The protective layer can be, for example, what is known as a release paper which can consist of a paper layer coated with silicone, but can also be made of another material with release characteristics, for example a packing material. It is also possible to use other fastening means, such as hook-and-loop fasteners or friction fastening.

The surface layer 13 can consist of any conventional material, for example one or more non-wovens, one or more plastic films, perforated non-woven, perforated plastic film, laminates of the materials just mentioned above or combinations thereof.

The backing layer 14 suitably consists of an essentially liquid-impermeable material such as, for example, a thin liquid impermeable plastic film, or of a material which is in itself liquid permeable but has been coated with a layer of plastic, resin or some other liquid impermeable material. The backing layer 14 can therefore consist of any material which satisfies the criterion of liquid-impermeability so that leakage from the underside is prevented and has sufficient flexibility and skin-friendliness for the purpose. Examples of suitable materials are plastic films made of polyethylene, polyester or polypropylene, foams, non-wovens and laminates of non-wovens and plastic films. A backing layer which consists of a laminate made of a liquid-impermeable plastic layer facing the absorbent body and a non-woven facing the underwear of the wearer provides a leakproof barrier layer with a textile feel. It is also possible to design the backing of the panty liner with a breathable backing layer made of, for example, SMS (spunbond-meltblown-spunbond non-woven) or of a breathable plastic film made of, for example, polyethylene.

The absorbent body 15 is suitably made from natural fibres such as, for example, cellulose pulp, absorbent synthetic fibres or mixtures of natural fibres and synthetic fibres. The absorbent body 15 preferably consists of an airlaid cellulose body. It is also possible to mix what are known as superabsorbents into the absorbent body. A superabsorbent is a polymer which is capable of absorbing several times its own weight of liquid. The absorbent body 15 can also contain additional components such as shape-stabilizers, liquid-spreading means or binders. It is also possible to use various types of absorbent foamed material in the absorbent body. The absorbent body can of course be constructed from one or more different layers; at least one admission layer, for example, can also be included in the absorbent body. In the following figures, the absorbent body and the admission layer are described separately, but this is only to indicate a number of different embodiments which are possible within the scope of the invention and is not intended to limit the definition of absorbent body to a body without an admission layer.

An admission layer 17 is arranged between the surface layer 13 and the absorbent body 15. The purpose of the admission layer 17 is to draw liquid into the absorbent article, spread the liquid and transport the liquid down to the absorbent body 15. The admission layer 17 can be made of, for example, a low-density non-woven material, a wadding or the like.

Figure 2b shows part of a section of another embodiment. The section shows the surface layer 13, the backing layer 14 and also the admission layer 17 and the absorbent body 15. The admission layer 17 is located between the surface layer 13 and the absorbent body 15. The absorbent body 15 is located between the admission layer 17 and the backing layer 14. The panty liner in Figure 2b has essentially cone-shaped cavities 20 which, in this embodiment, extend all the way through the absorbent body 15 and all the way through the admission layer 17. The essentially cone-shaped cavities 20 have a tip 21 and a base 22, the cavity having a smaller circumference at the tip 21 than at the base 22. In Figure 2b, the tip 21 is located closer to the surface layer 13 than the base 22. The cone-shaped cavities 20 also each have an inner wall 23 which consists entirely of the backing layer 14.

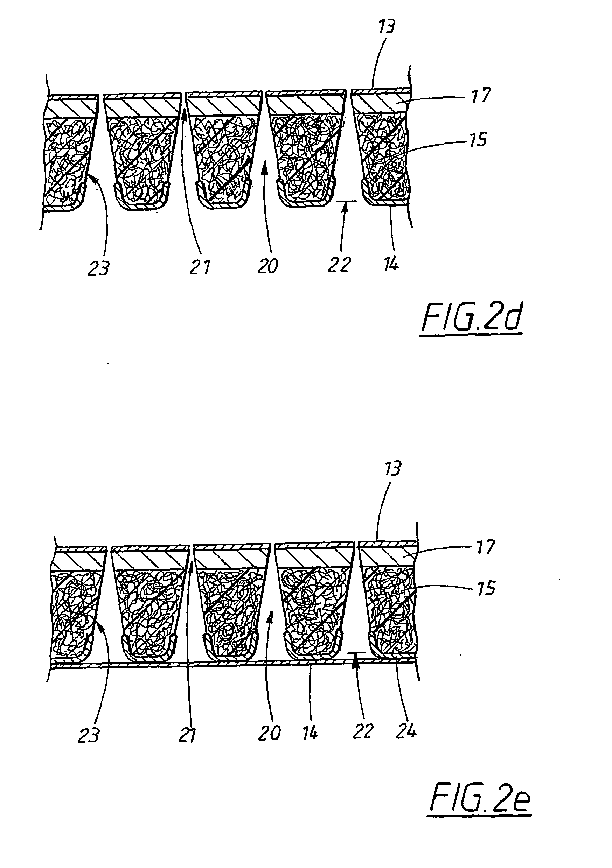

Figure 2d shows part of a section of a further embodiment of a panty liner. The section shows the surface layer 13, the backing layer 14 and also the admission layer 17 and the absorbent body 15. The admission layer 17 is located between the surface layer 13 and the absorbent body 15. The absorbent body 15 is located between the admission layer 17 and the backing layer 14. The panty liner in Figure 2d has essentially cone-shaped cavities 20 which, in this embodiment, extend all the way through the absorbent body 15 and all the way through the admission layer 17 and also through the surface layer 13. The essentially cone-shaped cavities 20 have a tip 21 and a base 22, the circumference of the cavities 20 being smaller at the tip 21 than at the base 22. The cone-shaped cavities 20 also each have an inner wall 23 or envelope surface which consists partly of the backing layer 14 and also of the absorbent body and to a certain extent the admission layer 17 and the surface layer 13.

Figure 2e shows part of a section of a further embodiment of a panty liner. The section shows the surface layer 13, a first backing layer 24 and also the admission layer 17 and the absorbent body 15. The admission layer 17 is located between the surface layer 13 and the absorbent body 15. The absorbent body 15 is located between the admission layer 17 and the first backing layer 24. The panty liner in Figure 6 also has essentially cone-shaped cavities 20 which, in this embodiment, extend all the way through the absorbent body 15 and all the way through the admission layer 17 and also through the surface layer 13. The essentially cone-shaped cavities 20 have a tip 21 and a base 22, the tip 21 being smaller than the base 22. The cone-shaped cavities 20 also each have an inner wall 23 or envelope surface which consists partly of the first backing layer 24 and also of the absorbent body and to a certain extent the admission layer 17 and the surface layer 13. The panty liner in Figure 2e also has a second, outer backing layer 14 which is located against the first backing layer 24 so that the essentially cone-shaped cavities 20 are not visible, or are not immediately conspicuous. The first backing layer 24 and the second backing layer 14 can consist of the same material but can also consist of different materials. Preferably, at least the second backing layer 14 is made of an essentially breathable material.

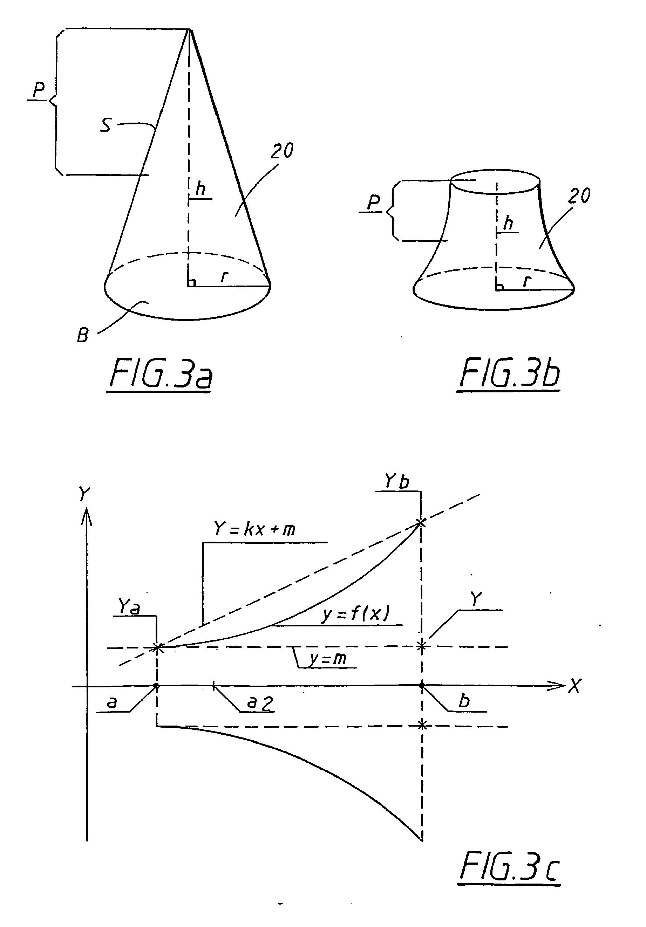

It is possible to describe the essentially cone-shaped cavity 20 using, for example, an ordinary cone according to Figure 3a with a height h, a base radius r, a side s, a base area $B = \pi \cdot r^2$, an envelope surface $M = \pi \cdot r \cdot s$ and a volume $V = (\pi \cdot r^2 \cdot h)/3$, and also a tip part P.

Figure 3b shows an alternative embodiment of an essentially cone-shaped cavity. The tip part P is blunted and has a flat surface opposite the base. This shape is of course applicable to pyramid-shaped cavities.

According to another embodiment, it is possible to describe all or part of the essentially cone-shaped hole by rotating a function f(X) around the X axis in an X-Y diagram as shown in Figure 3c. The volume V is then obtained by the formula:

$$V = \int_a^b \pi \cdot Y^2 \cdot dX = \pi \int_a^b f^2(X) \cdot dX$$

This applies when $Y_a < Y_b$, that is to say when $f(X_a) < f(X_b)$, that is to say the value Y of the function at the point a should be less than the value Y of the function at the point b, and also $a \leq a_2 < b$.

In addition to this, the function $Y = f(X)$ which describes the essentially cone-shaped hole should be such that values for Y should be $Y \leq kX + m$ in the interval X = a and X = b, and also the function $Y = f(X)$ should be such that values for Y should be $Y > m$ in the interval X = a and X = b (a defines the tip and b the base of the cone).

Where $Y = kX + m$ is the equation of the straight line, k is the inclination of the line and m is the intersection with the Y axis when X = 0. On rotation of $Y = kX + m$ in the interval X = a and X = b and with a < b and m = 0, the ordinary cone shown in Figure 3a is described.

According to another embodiment, the essentially cone-shaped hole can have a more funnel-like shape. The essentially cone-shaped hole is then described by values for Y having to be Y = m in the interval X = a and $X = a_2$, and the remaining part is described by the function $Y = f(X)$ having to be such that values for Y should be $Y \leq kX + m$ in the interval $X = a_2$ and X = b and the function $Y = f(X)$ having to be such that values for Y should be $Y > m$ in the interval X = $a_2$ and X = b and a < $a_2$ < b.

It should be noted that the Y axis corresponds to the longitudinal and transverse planes of the absorbent article and that the X axis corresponds to the thickness of the absorbent article, that is to say the distance between the liquid permeable surface layer and the essentially liquid impermeable backing layer of the absorbent article.

In one embodiment, the cavities have a height of roughly 0.5-30 mm, preferably 0.5-5 mm. The essentially cone-shaped cavities can have a base radius of roughly 0.5-10 mm, preferably 0.5-5 mm, and most preferably 0.5-2 mm.

In some embodiments, it may be advantageous to describe the base area instead of a radius, especially when a pyramid or a funnel-like cavity is described. The base area of the cavities is then roughly 0.78-315 mm$^2$, preferably 0.78-79 mm$^2$, and most preferably 0.78-51 mm$^2$.

Example

A panty liner according to the invention was manufactured with the following construction, in order: a surface layer made of carded non-woven with a basis weight of 26 g/m$^2$. The surface layer was attached using a hot-melt adhesive with a basis weight of 7 g/m$^2$ to a distribution ply in the form of an airlaid non-woven paper with a basis weight of 80 g/m$^2$ which was in turn attached using a hot-melt adhesive with a basis weight of 9 g/m$^2$ to an absorbent body made of chemithermomechanical pulp (CTMP) with a basis weight of 200 g/m$^2$. Superabsorbents (SAPs) with a basis weight of 50 g/m$^2$ were mixed into the absorbent body. The absorbent body was attached using a hot-melt adhesive with a basis weight of 5 g/m$^2$ to a polypropylene non-woven with a basis weight of 30 g/m$^2$.

The entire article was cold-needte-perforated in order to produce essentially cone-shaped cavities according to the invention. A layer of spunbond non-woven with a basis weight of 22 g/m$^2$ was then applied as backing layer. A hot-melt adhesive with a basis weight of 35 g/m$^2$ was subsequently applied to the backing layer, after which a release paper (protective paper for the fastening adhesive) with a basis weight of 43 g/m$^2$ was applied over the adhesive.

## Claims

1. Absorbent article having a first essentially liquid permeable surface layer (13), an essentially liquid impermeable backing layer (14) and, located between said liquid permeable surface layer (13) and said liquid impermeable backing layer (14), an absorbent body (15) comprising at least one layer, the absorbent article having cavities (20) which are essentially cone-shaped or have a pyramid-like or funnel-like shape, including those cavities having more sides than a pyramidal shape, said cavities (20) having a tip part (21) and a base (22), the tip part (21) having a smaller circumference than the base (22) and being located towards the liquid permeable surface layer (13) and the base (22) being located away from the liquid permeable surface layer (13), **characterized in that** the cavities (20) extend through the whole of the absorbent body (15).

2. Absorbent article according to any one of the preceding claims, the absorbent body (15) having cavities (20) of different sizes.

3. Absorbent article according to any one of the preceding claims, the envelope surface (23) of the cavities (20) consisting entirely or partly of the backing layer (14; 24).

4. Absorbent article according to any one of the preceding claims, the envelope surface (23) of the cavities (20) consisting entirely or partly of a separate layer (24) located between the absorbent body (15) and the liquid impermeable backing layer (14).

5. Absorbent article according to any one of the preceding claims, the envelope surface (23) of the cavities (20) consisting entirely or partly of a plastic film (24) located between the absorbent body (15) and the liquid impermeable backing layer (14).

6. Absorbent article according to any one of the preceding claims, the envelope surface (23) of the cavities (20) being at least partly hydrophobic.

7. Absorbent article according to any one of the preceding claims, the backing layer (14) being treated with one or more agents in order to change the characteristics of the backing layer, the agent(s) being for pH control, in order to provide a more hydrophobic surface, in order to provide a less hydrophobic surface or a hydrophilic surface, in order to provide an indication of temperature, moisture content, pH, enzyme activity, odour inhibition or in order to add bacteria to the article, for example lactobacteria.

8. Absorbent article according to any one of the preceding claims, the cavities (20) having a height of roughly 0.5-30 mm, preferably 0.5-5 mm, and a base area of roughly 0.19-79 mm$^2$, preferably 0.19-40 mm$^2$, and most preferably 0.19-4 mm$^2$.

9. Absorbent article according to any one of the preceding claims, it being possible to describe the volume of the cavities by rotating a function Y = f(X) around the X axis in an X-Y diagram, the volume V being obtained by the formula:

$$V = \int_a^b \pi \cdot Y^2 \cdot dX = \pi \int_a^b f^2(X) \cdot dX$$

when a < b, in addition to which the function Y = f(X) which describes the essentially cone-shaped hole should be such that values for Y should be Y ≤ kX + m in the interval X = a and X = b, and also the function Y = f(X) should be such that values for Y should be Y > m in the interval X = a and X = b, where Y = kX + m is the equation of the straight line, k is the inclination of the line and m is the intersection with the Y axis when X = 0, and a defines the tip and b the base of the cone.

**Patentansprüche**

1. Absorbierender Gegenstand mit einer ersten im Wesentlichen flüssigkeitsdurchlässigen Oberflächenlage (13), einer im Wesentlichen flüssigkeitsundurchlässigen Decklage (14) und einem zwischen der flüssigkeitsdurchlässigen Oberflächenlage (13) und der flüssigkeitsundurchlässigen Decklage (14) angeordneten Absorptionskörper (15), der wenigstens eine Lage umfasst, wobei der absorbierende Gegenstand Hohlräume (20) aufweist, die im Wesentlichen kegelförmig sind oder eine pyramidenähnliche oder trichterähnliche Form aufweisen, umfassend solche Hohlräume, die mehr Seiten als eine Pyramide aufweisen, und wobei die Hohlräume (20) einen oberen Teil (21) und eine Basis (22) aufweisen, der obere Teil (21) einen kleineren Umfang aufweist als die Basis (22) und in Richtung der flüssigkeitsdurchlässigen Oberflächenlage (13) angeordnet ist, und die Basis (22) entfernt von der flüssigkeitsdurchlässigen Oberflächenlage (13) angeordnet ist, **dadurch gekennzeichnet, dass** sich die Hohlräume (20) durch den gesamten Absorptionskörper (15) erstrecken.

2. Absorbierender Gegenstand nach einem der vorstehenden Ansprüche, bei dem der Absorptionskörper (15) Hohlräume (20) mit unterschiedlicher Größe aufweist.

3. Absorbierender Gegenstand nach einem der vorstehenden Ansprüche, bei dem die die Hohlräume (20) umschließende Fläche (23) vollständig oder teilweise aus der Decklage (14; 24) besteht.

4. Absorbierender Gegenstand nach einem der vorstehenden Ansprüche, bei dem die die Hohlräume (20) umschließende Fläche (23) vollständig oder teilweise aus einer separaten Lage (24) besteht, die zwischen dem Absorptionskörper (15) und der flüssigkeitsundurchlässigen Decklage (14) angeordnet ist.

5. Absorbierender Gegenstand nach einem der vorstehenden Ansprüche, bei dem die die Hohlräume (20) umschließende Fläche (23) vollständig oder teilweise aus einer Kunststofffolie (24) besteht, die zwischen dem Absorptionskörper (15) und der flüssigkeitsundurchlässigen Decklage (14) angeordnet ist.

6. Absorbierender Gegenstand nach einem der vorstehenden Ansprüche, bei dem die die Hohlräume (20) umschließende Fläche (23) wenigstens teilweise hydrophob ist.

7. Absorbierender Gegenstand nach einem der vorstehenden Ansprüche, bei dem die Decklage (14) mit einem oder mehreren Mitteln behandelt ist, um die Eigenschaften der Decklage zu ändern, wobei das Mittel/die Mittel zur pH-Steuerung dienen, um eine hydrophobere Fläche bereitzustellen, um eine weniger hydrophobe Fläche oder eine hydrophile Fläche bereitzustellen, um eine Anzeige der Temperatur, des Feuchtigkeitsgehalts, des pH-Werts, der Enzymaktivität oder eine Geruchsverminderung bereitzustellen oder um dem Gegenstand Bakterien zuzufügen, zum Beispiel Lactobakterien.

8. Absorbierender Gegenstand nach einem der vorstehenden Ansprüche, bei dem die Hohlräume (20) eine Höhe von ungefähr 0,5-30 mm, vorzugsweise 0,5-5 mm, und eine Basisfläche von ungefähr 0,19-79 mm$^2$, vorzugsweise 0,19-40 mm$^2$, und am meisten bevorzugt 0,19-4 mm$^2$ aufweisen.

9. Absorbierender Gegenstand nach einem der vorstehenden Ansprüche, bei dem es möglich ist, das Hohlraumvolumen durch Rotation einer Funktion Y=f(X), um die X-Achse in einem X-Y-Diagramm zu beschreiben, wobei das Volumen V durch die folgende Formel erzielt wird:

$$V = \int_a^b \pi \cdot Y^2 \cdot dX = \pi \int_a^b f^2(X) \cdot dX$$

wenn a < b, wobei die Funktion Y = f(X), die im Wesentlichen das kegelförmige Loch beschreibt, zusätzlich derart sein sollte, dass die Werte für Y im Intervall X = a und X = b Y $\leq$ kX + m sind, und ferner sollte die Funktion Y = f (X) derart sein, dass die Werte für Y im Intervall X = a und
X = b Y > m sein sollten, wobei Y = kX + m die Gleichung der geraden Linie ist, k die Neigung der Linie ist, und m der Schnitt mit der Y-Achse ist, wenn X = 0, und a die Spitze und b die Basis des Kegels definieren.

**Revendications**

1. Article absorbant comportant une première couche de surface (13) essentiellement perméable aux liquides, une couche de support (14) essentiellement imperméable aux liquides et, entre ladite couche de surface (13) perméable aux liquides et ladite couche de support (14) imperméable aux liquides, un corps absorbant (15) comprenant au moins une couche, l'article absorbant comportant des cavités (20) qui sont essentiellement de forme conique ou qui ont une forme de type pyramide ou de type entonnoir, y compris les cavités ayant plus de côtés qu'une forme pyramidale, lesdites cavités (20) ayant une partie d'extrémité (21) et une base (22), la partie d'extrémité (21) ayant une plus petite circonférence que la base (22) et étant située vers la couche de surface (13) perméable aux liquides et la base (22) étant située à l'écart de la couche de surface (13) perméable aux liquides, **caractérisé en ce que** les cavités (20) s'étendent dans tout le corps absorbant (15).

2. Article absorbant selon l'une quelconque des revendications précédentes, le corps absorbant (15) comportant des cavités (20) de différentes tailles.

3. Article absorbant selon l'une quelconque des revendications précédentes, la surface d'enveloppe (23) des cavités (20) étant constituée entièrement ou partiellement de la couche de support (14 ; 24).

4. Article absorbant selon l'une quelconque des revendications précédentes, la surface d'enveloppe (23) des cavités (20) étant constituée entièrement ou partiellement d'une couche distincte (24) située entre le corps absorbant (15) et la couche de support (14) imperméable aux liquides.

5. Article absorbant selon l'une quelconque des revendications précédentes, la surface d'enveloppe (23) des cavités (20) étant constituée entièrement ou partiellement d'un film en plastique (24) situé entre le corps absorbant (15) et la couche de support (14) imperméable aux liquides.

6. Article absorbant selon l'une quelconque des revendications précédentes, la surface d'enveloppe (23) des cavités (20) étant au moins partiellement hydrophobe.

7. Article absorbant selon l'une quelconque des revendications précédentes, la couche de support (14) étant traitée avec un ou plusieurs agent(s) pour modifier les caractéristiques de la couche de support, le ou les agent(s) servant à réguler le pH, afin de fournir une surface plus hydrophobe, afin de fournir une surface moins hydrophobe ou une surface hydrophile, afin de fournir une indication de température, de teneur en humidité, de pH, d'activité enzymatique, de neutralisation des odeurs ou afin d'ajouter des bactéries à l'article, par exemple des lactobactéries.

8. Article absorbant selon l'une quelconque des revendications précédentes, les cavités (20) ayant une hauteur d'environ 0,5 à 30 mm, de préférence de 0,5 à 5 mm, et une aire de base d'environ 0,19 à 79 mm$^2$, de préférence de 0,19 à 40 mm$^2$, et mieux encore de 0,19 à 4 mm$^2$.

9. Article absorbant selon l'une quelconque des revendications précédentes, dans lequel le volume des cavités peut être décrit en effectuant une rotation d'une fonction Y = f(X) autour de l'axe des X dans un repère X-Y, le volume V étant donné par la formule :

$$V = \int_a^b \pi \cdot Y^2 \cdot dX = \pi \int_a^b f^2(X) \cdot dX$$

quand a < b, la fonction Y = f(X) qui décrit le trou de forme essentiellement conique devant en outre respecter les conditions selon lesquelles Y ≤ kX + m dans l'intervalle X = a et X = b, de plus la fonction Y = f(X) doit être telle que les valeurs de Y doivent respecter Y > m dans l'intervalle X = a et X = b, où Y = kX + m est l'équation de la droite, k est la pente de la droite et m est l'intersection avec l'axe des Y quand X = 0, et a définit le sommet et b la base du cône.

*FIG.1*

_FIG.2a_

_FIG.2b_

_FIG.2c_

*FIG.2d*

*FIG.2e*

FIG.3a

FIG.3b

FIG.3c

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1040800 A1
- WO 0010500 A
- EP 0518340 A1